# EUROPEAN PATENT SPECIFICATION

(11) **EP 4 477 179 B1**
(45) Date of publication and mention of the grant of the patent: **01.04.2026**
(21) Application number: 22933435.4
(22) Date of filing: 24.03.2022
(51) Int. Cl.: A61B 46/10, A61B 34/30

(54) **DRAPE ATTACHMENT STRUCTURE FOR SURGERY ASSIST APPARATUS, AND DRAPE**
ABDECKTUCHBEFESTIGUNGSSTRUKTUR FÜR EINE CHIRURGISCHE HILFSVORRICHTUNG UND ABDECKTUCH
STRUCTURE DE FIXATION DE CHAMP POUR APPAREIL D'ASSISTANCE CHIRURGICALE, ET CHAMP

(43) Date of publication of application: 18.12.2024
(73) Proprietor: RIVERFIELD Inc., 1070052 Tokyo (JP)
(72) Inventor: YAMAMOTO, Nobuaki, Tokyo 160-0017 (JP); KISHIMOTO, Rikiya, Tokyo 160-0017 (JP); MORIKAWA, Toshiaki, Tokyo 165-0022 (JP)
(74) Representative: Adares PartGmbB
(86) International application number: PCT/JP2022/014133
(87) International publication number: WO 2023/181295

(56) References cited:
- EP-B1- 2 554 105
- WO-A1-2011/121695
- WO-A1-2011/121695
- JP-A- 2003 325 543
- JP-A- 2006 061 272
- JP-A- 2006 061 272
- KR-A- 20110 036 452
- US-A1- 2015 202 009
- US-A1- 2015 202 009
- US-A1- 2021 059 657

## Description

### Technical Field

The present disclosure relates to a technical field about a drape attachment structure for a surgery assist apparatus including a plurality of coupling arms and about a drape to be used for the surgery assist apparatus.

### Background Art

In recent years, surgical operations using a surgery assist apparatus have been gaining popularity. Such a surgery assist apparatus includes a plurality of coupling arms that are coupled together in order pivotably or rotatably, the coupling arms are caused to pivot by driving forces of actuators or the like, and one of the coupling arms located at one end in a coupling direction is provided with a holder configured to hold a surgical instrument. To the holder, a surgical instrument including a portion (distal end portion) to be inserted into a body cavity of a patient is attached via a separator and an adapter, thus being held by the holder. As the surgical instrument, an endoscope, forceps, or the like is used in accordance with a type or conditions of an operation.

In the surgery assist apparatus, when the plurality of coupling arms are caused to pivot or rotate in their predetermined directions, a position and an attitude of the surgical instrument change with movements of the coupling arms, and a surgical operation is performed with the surgical instrument inserted into the body cavity of the patient.

A surgical operation with the surgery assist apparatus as described above is typically performed by a surgeon (doctor) remotely operating the surgery assist apparatus installed in an operating room in a primary/replica architecture. For performing such a surgical operation, a pivot point serving as a reference point for a position and an attitude of a surgical instrument is set.

The pivot point is at a position substantially coinciding with a position of a port that is formed in a body cavity of a patient and through which the surgical instrument is inserted, and in a case where a trocar is used, the position of the pivot point is made to substantially coincide with a position of the trocar. Therefore, in a state where the surgical instrument is inserted into the body cavity of the patient, the position and the attitude of the surgical instrument are controlled such that a portion of the surgical instrument always passes through the pivot point, and the portion of the surgical instrument passing through the pivot point prevents occurrence of a load on tissue in a vicinity of a body surface of the patient, thus ensuring safety.

In an operation, the surgery assist apparatus as described above is covered with a sterilized drape (e.g., see Patent Literature 1). The drape includes a transparent cover formed in a film form and a hood attached to the cover. In Patent Literature 1, the cover is shown as a drape body, and the hood is shown as a mount cover.

In the surgery assist apparatus, its holder and apparatus body are covered with the cover with the hood being attached to a portion of the holder, so that a sterilized state is kept. Therefore, an inside of the drape is regarded as a sanitary area and divided from an unsanitary area that is an outside of the drape.

The hood is formed with a penetration hole, and a separator and an adapter are attached to the hood from the unsanitary area side. To the separator and the adapter, the surgical instrument is attached, and the surgical instrument is attached to the holder via the separator and the adapter.

By attaching the separator and the adapter to the hood and attaching the surgical instrument to the separator and the adapter in this manner, electrical connection and mechanical joining are made between the surgical instrument and the holder via the penetration hole formed in the hood.

Patent Literature 2 describes a robotic surgical system having a sterile tool holder, a robotic arm, an interface thereof, a sterile adapter, a sterile drape and a sterile adapter to be mounted on the interface. The sterile adapter can have tabs/clips to engage grooves on the interface of the robot arm, wherein after the sterile adapter is attached to the robot interface, the sterile tool holder can be attached to the robot and can include one or more positioning members such as pins or pegs that can pierce the sterile drape. The tool holder can be attached to the robot using a tightening screw which when tightened, pieces the sterile drape.

Patent Literature 3 describes a drape which can have perforations to enable piercings of the drape.

Patent Literature 4 describes a probe and an imaging apparatus for diagnosis having a connection end-surface cover 36 which can be broken along perforations by the press force thereof.

Patent Literatures 5 describes a medical instrument holding device which can selectively hold a plurality of surgical instruments and is equipped with an arm rack, a sterilized drape, a pre-sterilized first adapter, a pre-sterilized second adapter, and a pre-sterilized surgical instrument position-adjusting mechanism. The drape is attachable to/detachable from the arm rack. The first adapter can be attached to/detached from the arm rack while keeping the state of sterilization from outside of the drape. The second adapter is attachable to/detachable from the first adapter, and has a pre-sterilized surgical instrument holding section for holding a surgical instrument.

Patent Literature 6 describes a surgical robot and a sterile drape covering the surgical robot to cover a plurality of robot arms. A sterile drape has a fixing part that is attached to the robot.

### Citation List

### Patent Literature

Patent Literature 1: JP 2021-171345 A
Patent Literature 2: US 2015/202009 A1
Patent Literature 3: US 2021/059657 A1
Patent Literature 4:EP 2 554 105 B1 & WO 2011/121695 A1
Patent Literature 5: JP 2006 061272 A
Patent Literature 6: KR 2011 0036452 A

### Summary of Invention

### Technical Problem

In the surgery assist apparatus, the hood is formed with the penetration hole for making the electrical connection and the mechanical joining between the surgical instrument and the holder, and thus, for example, in the attachment of the separator and the adapter to the holder, there is a risk that a finger or the like of a worker may be mistakenly inserted into the penetration hole to come into contact with the holder.

If such contact of the finger or the like with the holder occurs, the sanitary area cannot be kept.

Thus, a drape attachment structure for a surgery assist apparatus and a drape according to the present invention have an object to keep the sanitary area that is an area where the holder and the apparatus body are present.

### Solution to Problem

The problem is solved by a drape according to claim 1 and a drape attachment structure according to claim with a drape according to claim 1, further embodiments are disclosed in the dependent claims. The drape attachment structure for a surgery assist apparatus according to the present disclosure is a drape attachment structure for a surgery assist apparatus that includes an apparatus body including a plurality of coupling arms and includes a holder supported by the apparatus body, a separator being made attachable to or detachable from the holder, the holder and the apparatus body being covered with a drape in an operation, wherein the drape includes a cover and a hood, the cover being in a film form, the hood being to be attached to one end portion of the holder and being formed with a penetration hole, the separator includes a mounting portion and a penetrating portion, the mounting portion being capable of being in close contact with the hood, the penetrating portion protruding from the mounting portion, the hood includes a closure sheet that closes the penetration hole in a state before the separator is attached to the holder, and the separator is attached to the holder in a state where the penetrating portion breaks through the closure sheet to penetrate into the penetration hole, and the penetration hole is closed by the mounting portion.

Thus, the penetration hole of the hood is closed by the closure sheet in a state before the separator is attached to the holder, and the penetration hole is closed by the mounting portion in a state where the separator is attached to the holder.

The drape according to the present disclosure is a drape to be used for a surgery assist apparatus that includes an apparatus body including a plurality of coupling arms and includes a holder supported by the apparatus body, a separator being made attachable to or detachable from the holder, the separator including a mounting portion and a penetrating portion, the holder and the apparatus body being covered with the drape in an operation, the drape including: a cover and a hood, the cover being in a film form, the hood being to be attached to one end portion of the holder and being formed with a penetration hole, wherein the hood includes a closure sheet that closes the penetration hole in a state before the separator is attached to the holder, and at a time when the separator is attached to the holder, the closure sheet is broken through by the penetrating portion, and the penetration hole is closed by the mounting portion in a state where the separator is attached to the holder.

Thus, the penetration hole of the hood is closed by the closure sheet in a state before the separator is attached to the holder, and the penetration hole is closed by the mounting portion in the state where the separator is attached to the holder.

### Advantageous Effect of Invention

According to the present invention, the penetration hole of the hood is closed by the closure sheet in a state before the separator is attached to the holder, and the penetration hole is closed by the mounting portion in a state where the separator is attached to the holder, so that it is possible to keep a sanitary area that is an area where the holder and the apparatus body are present.

### Brief Description of Drawings

FIG. 1 illustrates, together with FIG. 2 to FIG. 9, an embodiment of a drape attachment structure for a surgery assist apparatus, and a drape according to the present invention, and FIG. 1 is a schematic perspective view illustrating the surgery assist apparatus in a usage state.
FIG. 2 is a schematic perspective view of the surgery assist apparatus.
FIG. 3 is an exploded perspective view of a holder.
FIG. 4 is a perspective view illustrating the holder and an apparatus body that are covered with the drape, together with a separator.
FIG. 5 is a plan view of the drape.
FIG. 6 is a sectional view illustrating a hood and the like.
FIG. 7 is a perspective view illustrating the hood and the like.
FIG. 8 is a sectional view illustrating a state before the separator is attached to the holder.
FIG. 9 is a sectional view illustrating a state where the separator is attached to the holder via the hood.

### Description of Embodiment

An embodiment for carrying out a drape attachment structure for a surgery assist apparatus, and a drape according to the present invention will be described below with reference to the accompanying drawings.

The embodiment described below shows an example in which the surgery assist apparatus used in the present invention is applied to a surgery assist apparatus of a type that is fixed to an operating table in its use. Note that the scope of application of the surgery assist apparatus used in the present invention is not limited to the surgery assist apparatus of the type fixed to an operating table in its use and can be also applied to a surgery assist apparatus of a type that is installed on a floor or the like of an operating room in its use or a surgery assist apparatus of a type that is attached to a ceiling or wall surface of an operating room in its use.

Note that front-back, up-down, and left-right directions to be used in the following description are for convenience of description only and not intended to limit the practice of the present invention.

### <Schematic Configuration of Surgery Assist Apparatus, etc.>

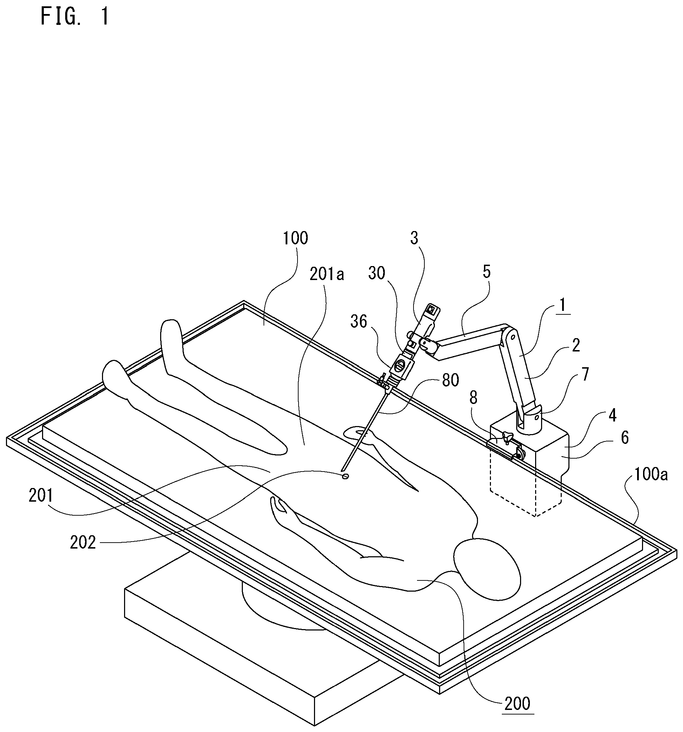

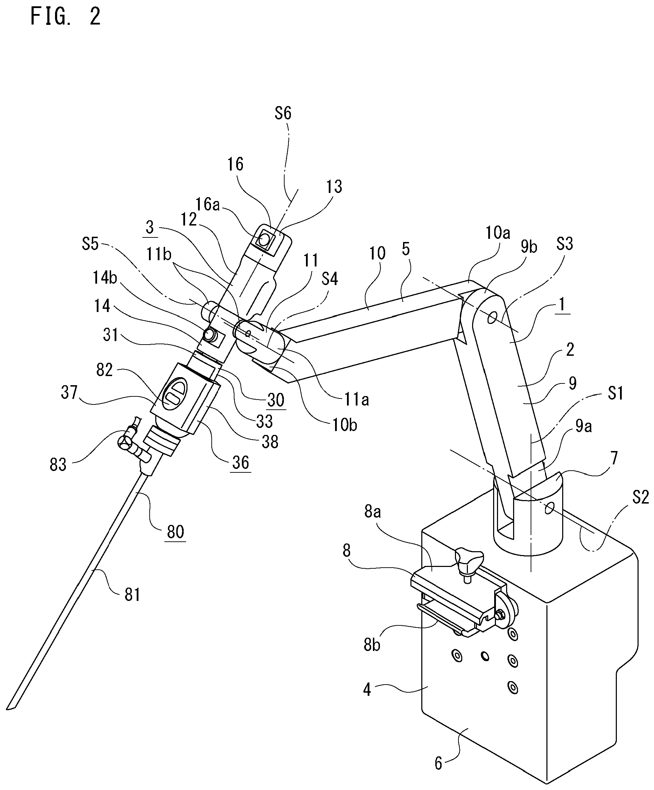

First, a schematic configuration of a surgery assist apparatus 1 and the like will be described (see FIG. 1 and FIG. 2).

In an operating room, an operating table 100 is installed, and on the operating table 100, a patient 200 is laid in, for example, a supine position. The operating table 100 is provided with a fixing rail 100a on a side portion of the operating table 100. The patient 200 is formed with a port 202 in a body cavity 201 of the patient 200, such as on an abdominal wall 201a. Through the port 202, a portion (a distal end portion) of a surgical instrument described later is inserted when a surgical operation is performed. The port 202 is a small hole through which the portion of the surgical instrument is inserted.

The surgery assist apparatus 1 includes an apparatus body 2 and a holder 3. Inside the apparatus body 2, for example, one electric motor not illustrated and a plurality of pneumatic actuators not illustrated are disposed.

The apparatus body 2 includes a fixation base 4 that is made fixable to the operating table 100 and an arm body 5 that is coupled to the fixation base 4.

The fixation base 4 includes a base body 6 that has a substantially rectangular parallelepiped shape and inside which predetermined mechanisms and the like are provided, a pedestal 7 that is rotatably supported by the base body 6, and a clamper 8 that protrudes from the base body 6. The clamper 8 includes an upper clamp part 8a and a lower clamp part 8b, and at least one of the upper clamp part 8a and the lower clamp part 8b is configured to move in directions of separating from and approaching the other.

From a surface of the base body 6 opposite to a surface of the base body 6 on which the clamper 8 is provided, an air suction pipe not illustrated protrudes. The air suction pipe is connected to a compressor via a suction tube. Therefore, an inside of the fixation base 4 is supplied with compressed air from the compressor. The compressed air supplied to the inside of the fixation base 4 passes through the inside of the fixation base 4 to an inside of the arm body 5, to be used as driving forces for moving parts of the arm body 5.

The surgery assist apparatus 1 is fixed to the operating table 100 by the upper clamp part 8a and the lower clamp part 8b of the clamper 8 sandwiching the fixing rail 100a in an up-down direction.

Here, for example, the surgery assist apparatus 1 is conveyed to a sterilization room by a carriage (stand) not illustrated with the fixation base 4 attached to the carriage, is subjected to sterilization in the sterilization room, and is conveyed to the operating room to be used in an operation.

The pedestal 7 is rotatably supported by the base body 6 about an axis S1 that extends in a vertical direction, and a portion of the pedestal 7 is located above an upper surface of the fixation base 4. The pedestal 7 is caused to rotate with respect to the fixation base 4 by, for example, a driving force of an electric motor.

The arm body 5 includes a first coupling arm 9, a second coupling arm 10, and a third coupling arm 11. Note that the number of coupling arms of the arm body 5 is not limited to three, is only required to be more than one, and may be two or more than three.

The first coupling arm 9 is provided with one end portion in its longitudinal direction as a first coupling portion 9a and is provided with the other end portion in the longitudinal direction as a second coupling portion 9b. With the first coupling portion 9a being coupled to the pedestal 7, the first coupling arm 9 is made pivotable with respect to the pedestal 7 about an axis S2 that extends in a horizontal direction. The first coupling arm 9 is caused to pivot with respect to the pedestal 7 by, for example, a pneumatic actuator that is operated with the compressed air supplied by the compressor.

The second coupling arm 10 is provided with one end portion in its longitudinal direction as a first coupling portion 10a and is provided with the other end portion in the longitudinal direction as a second coupling portion 10b. With the first coupling portion 10a being coupled to the second coupling portion 9b of the first coupling arm 9, the second coupling arm 10 is made pivotable with respect to the first coupling arm 9 about an axis S3 that extends in the horizontal direction. The second coupling arm 10 is caused to pivot with respect to the first coupling arm 9 by, for example, a pneumatic actuator that is operated with the compressed air supplied by the compressor.

The third coupling arm 11 is provided with one end portion in its longitudinal direction as a first coupling portion 11a and is provided with the other end portion in the longitudinal direction as a second coupling portion 11b. The second coupling portion 11b includes, for example, two arm parts. With the first coupling portion 11a being coupled to the second coupling portion 10b of the second coupling arm 10, the third coupling arm 11 is made rotatable with respect to the second coupling arm 10 about an axis S4 that extends in a direction perpendicular to the axis S3. The third coupling arm 11 is, for example, a component that performs free rotational motion; the third coupling arm 11 is not caused to rotate by an external driving force, such as a driving force of an electric motor or a pneumatic actuator, but is caused to rotate by its own weight or in response to another motion of the first coupling arm 9, the second coupling arm 10, and the like.

### <Specific Configuration of Holder, etc.>

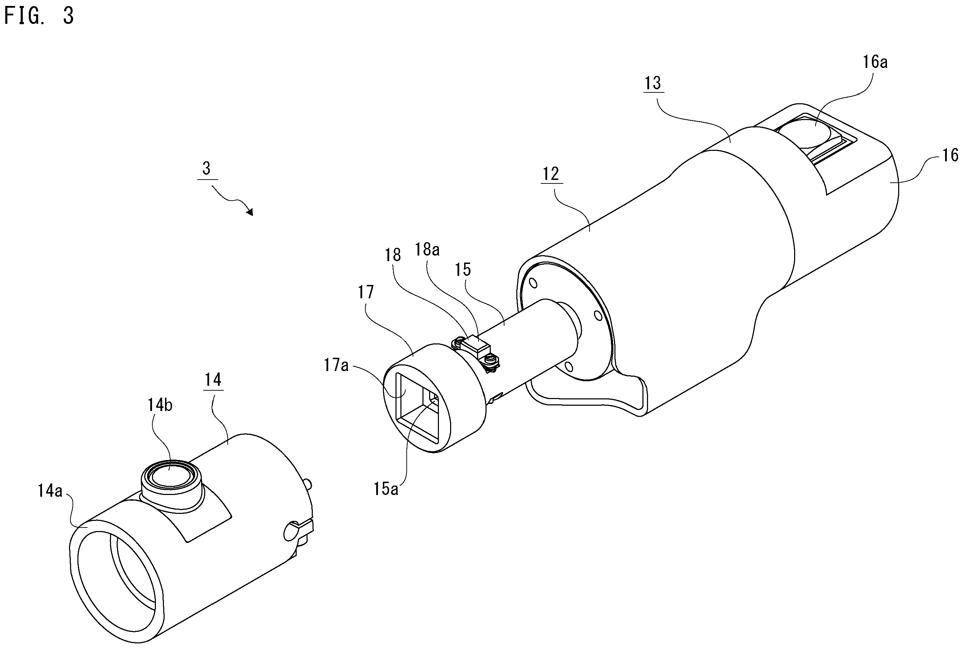

Next, a specific configuration of the holder 3 and the like will be described (see FIG. 3).

The holder 3 is pivotably supported by (coupled to) the second coupling portion 11b of the third coupling arm 11. The holder 3 has an external shape formed in a substantially columnar shape and is coupled to the second coupling portion 11b at its middle portion in its longitudinal direction (axial direction). The holder 3 is made pivotable with respect to the third coupling arm 11 about an axis S5 that extends in a direction perpendicular to the axis S4. The holder 3 is, for example, a component that performs free pivotal motion; the holder 3 is not caused to rotate by an external driving force, such as a driving force of an electric motor or a pneumatic actuator, but is caused to pivot by its own weight or in response to another motion of the first coupling arm 9, the second coupling arm 10, the third coupling arm 11, and the like.

The holder 3 is made partially rotatable and includes a base cylindrical part 12 that is not caused to rotate, a rotatable part 13 that is rotatably supported by the base cylindrical part 12, and a cylindrical coupling part 14 that is attached to the base cylindrical part 12.

The base cylindrical part 12 is formed with a shaft support hole not illustrated that penetrates the base cylindrical part 12 in its axial direction.

The rotatable part 13 includes a shaft part 15 that partially penetrates into the shaft support hole of the base cylindrical part 12, an operation part 16 that is joined to one end portion of the shaft part 15, and a detachable part 17 that is joined to the other end portion of the shaft part 15. The rotatable part 13 is made rotatable with respect to the base cylindrical part 12 and the cylindrical coupling part 14 in a direction around an axis S6 that is perpendicular to the axis S5. For example, the rotatable part 13 is made rotatable by a pneumatic actuator operated with the compressed air supplied by the compressor and is also made manually rotatable.

To the shaft part 15, a lock mechanism 18 is attached, and a portion of the lock mechanism 18 protrudes outward from an outer circumferential surface of the shaft part 15. The portion of the lock mechanism 18 protruding from the outer circumferential surface of the shaft part 15 is provided as a depressible part 18a. The shaft part 15 is formed with an insertion hole 15a that extends in its axial direction.

In the operation part 16, a switch button 16a is disposed. The switch button 16a has a function of switching driving forces to rotate the rotatable part 13 from one to another.

The detachable part 17 has an outer circumferential surface formed in a circumferential surface and includes a joint recess 17a that opens in an axial direction of the detachable part 17. The joint recess 17a is formed in a rectangular shape. The joint recess 17a communicates with the insertion hole 15a of the shaft part 15, and a size of an opening of the joint recess 17a is made larger than a size of an opening of the insertion hole 15a.

The cylindrical coupling part 14 is formed in a substantially cylindrical shape and is attached to the base cylindrical part 12 with the shaft part 15 and the detachable part 17 being covered with the cylindrical coupling part 14. The cylindrical coupling part 14 is supported by (coupled to) the second coupling portion 11b of the third coupling arm 11. An end surface of the cylindrical coupling part 14 on an opposite side to the base cylindrical part 12 is formed as an abutting surface 14a.

In the cylindrical coupling part 14, an unlock button 14b is disposed. When the unlock button 14b is operated, the unlock button 14b depresses the depressible part 18a of the lock mechanism 18. Note that the depressible part 18a is depressed by the operation of the unlock button 14b only when the lock mechanism 18 is positioned in the same phase as the unlock button 14b in a rotation direction of the shaft part 15.

In the holder 3, while the switch button 16a is operated, the rotatable part 13 is made manually rotatable. In contrast, while the switch button 16a is not operated, the rotatable part 13 is made rotatable by a driving force of the pneumatic actuator.

By a user of the surgery assist apparatus 1, such as a doctor or an assistant, manually rotating the operation part 16, the shaft part 15 and the detachable part 17 are caused to rotate integrally, and the rotatable part 13 is caused to rotate with respect to the base cylindrical part 12 and the cylindrical coupling part 14.

As described above, since the surgery assist apparatus 1 is configured such that the components are rotatable or pivotable about the six axes of the axis S1 to the axis S6, the surgery assist apparatus 1 has high degrees of freedom in orientation and position (attitude) of a surgical instrument that is held by the holder 3, and thus it is possible to perform an operation speedily with high accuracy. In particular, since the free rotational motion or free pivotal motion is performed about the axis S4 or the axis S5, and the axis S4 and the axis S5 are perpendicular to each other, it is possible to reduce a load on tissue in a vicinity of a body surface of the patient 200 when an orientation or a position of the port 202 is changed by changing an orientation or a position of the surgical instrument inserted through the port 202, a breathing state of the patient 200, or the like.

In the surgery assist apparatus 1, rotation of the arm body 5 about the axis S1 at a position farthest from the surgical instrument is performed by the driving force of the electric motor, and a rotation or pivot of the arm body 5 about the axis S2, axis S3, or axis S6 at positions closer to the surgical instrument than the axis S1 is performed by the driving force of one of the pneumatic actuators.

Therefore, the driving forces of the pneumatic actuators that impose lighter loads on the patient 200 than the driving force of the electric motor perform operation of the arm body 5 at positions closer to the surgical instrument, so that it is possible to further reduce the load on the tissue in the vicinity of the body surface of the patient 200.

Furthermore, the number of locations where the arm body 5 is caused to rotate or pivot by the driving forces of the pneumatic actuators being made larger than the number of locations where the arm body 5 is caused to rotate by the driving force of the electric motor can serve to reduce the load on the tissue in the vicinity of the body surface of the patient 200.

Note that, in the surgery assist apparatus 1, an ultrasonic motor, which can perform positioning with high accuracy, such as a piezoelectric motor, may be used instead of the electric motor or one of the pneumatic actuators. Using the ultrasonic motor also enables power saving or downsizing of the surgery assist apparatus 1 to be achieved.

### <Configuration of Drape>

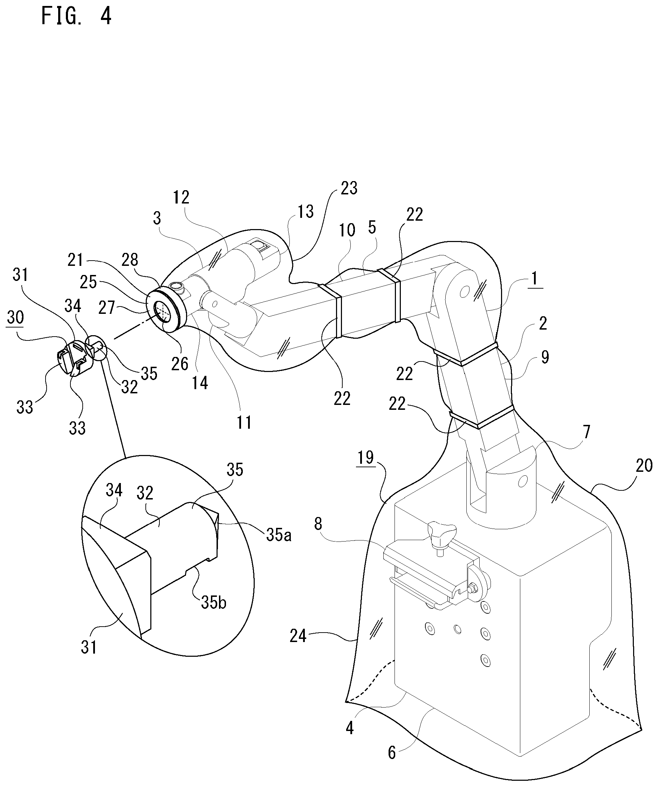

Next, a configuration of a drape 19 with which the apparatus body 2 and the holder 3 are covered will be described (see FIG. 4 to FIG. 7).

In an operation, the apparatus body 2 and the holder 3 in a sterilized state are covered with the drape 19 that has been sterilized (see FIG. 4).

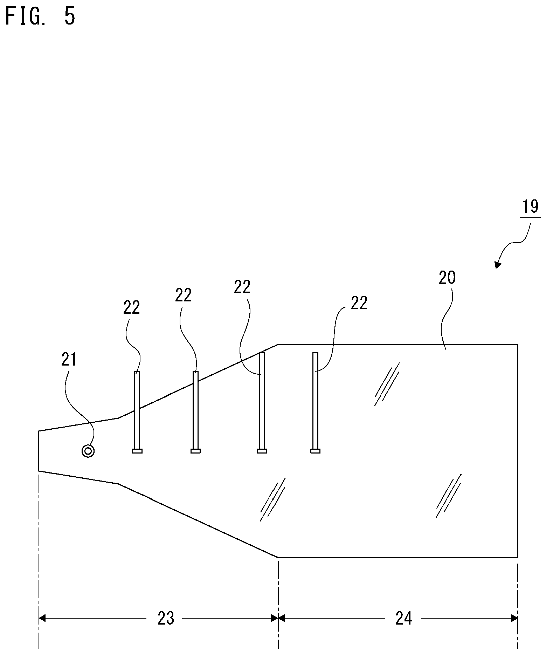

The drape 19 includes a cover 20 that is in a film form, a hood 21 that is attached to the cover 20, and a plurality of winding tapes 22 that are each partially stuck on the cover 20 by bonding or the like (see FIG. 5).

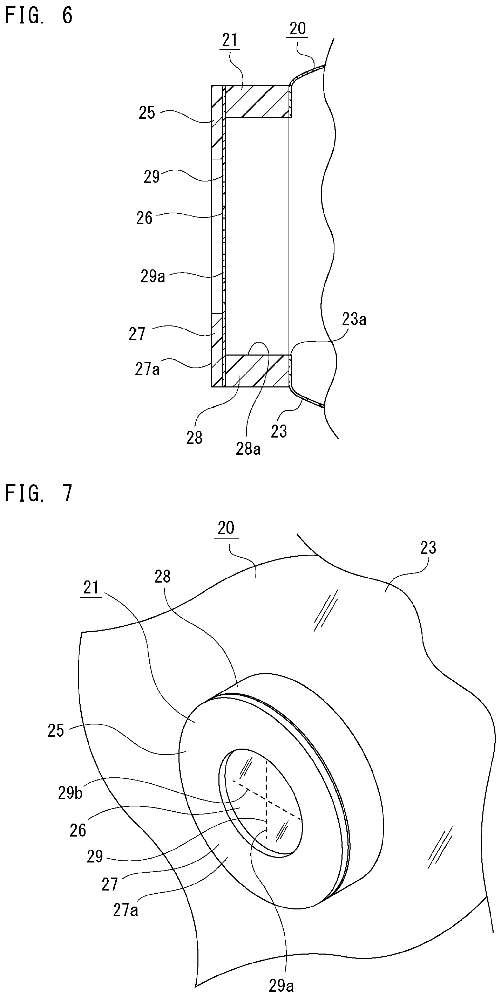

The cover 20 is formed of a transparent resin material, such as polyethylene, and is made to have such a size that the entire apparatus body 2 and holder 3 are covered. The cover 20 is formed in a bag shape, a first covering portion 23 that is a substantially half portion of the cover 20 on a distal end side, on which the holder 3 and the like are covered, is formed in such a shape that a diameter of the first covering portion 23 decreases as the first covering portion 23 extends toward its distal end, and a second covering portion 24 that is a substantially half portion of the cover 20 on a proximal end side is formed in a cylindrical shape having a diameter that is the same as a maximum diameter of the first covering portion 23. The first covering portion 23 is formed with a through hole 23a at a position close to the distal end (see FIG. 6).

The hood 21 includes a mounting base 25 that is mounted on one end portion (a distal end portion) of the holder 3 in its longitudinal direction and a closure sheet 26 that is held by the mounting base 25 (see FIG. 6 and FIG. 7). The mounting base 25 of the hood 21 is attached, from an outer surface side by bonding or the like, to a circumferential portion of the through hole 23a that is formed in the first covering portion 23.

The mounting base 25 includes a surface portion 27 having a small thickness and an annular shape and includes a cylindrical portion 28 having a cylindrical shape, and the surface portion 27 and the cylindrical portion 28 are joined together in a state of sandwiching the closure sheet 26 in an axial direction. A surface of the surface portion 27 is formed as a pressable surface 27a.

The surface portion 27 and the cylindrical portion 28 are formed of, for example, polyethylene foam material. Therefore, the mounting base 25 has elasticity (cushioning properties). The surface portion 27 and the cylindrical portion 28 are made to have the same outer diameter, and an inner diameter of the surface portion 27 is made smaller than an inner diameter of the cylindrical portion 28. A space inside the cylindrical portion 28 is formed as a penetration hole 28a.

The closure sheet 26 is formed in a circular shape having an outer diameter that is the same as the outer diameter of the surface portion 27 and is formed of, for example, a material that is the same as the material of the cover 20. The cover 20 and the closure sheet 26 being formed of the same material dispense with a need to use different materials to form the cover 20 and the closure sheet 26, and thus it is possible to reduce procurement costs of the material and to use the material effectively, thus serving to reduce production costs of the drape 19. Note that the cover 20 and the closure sheet 26 may be formed of different materials, and the closure sheet 26 may be formed of a non-transparent material such as a translucent or opaque material.

The closure sheet 26 is fixed, by bonding or the like, to the surface portion 27 and the cylindrical portion 28 with its outer circumferential portion being positioned between the surface portion 27 and the cylindrical portion 28, and is held by the mounting base 25 under a certain tension. The closure sheet 26 is formed with perforations 29 at a position corresponding to a portion inside the surface portion 27. For example, the perforations 29 are formed in a cross shape intersecting at a center of the penetration hole 28a and include a first linear part 29a and a second linear part 29b that are perpendicular to each other.

Note that the perforations formed in the closure sheet 26 may be formed in any pattern, and the perforations may include, for example, one linear part, three or more linear parts, or at least one curve part. Alternatively, the perforations may include at least one linear part and at least one curve part. The closure sheet 26 need not be formed with perforations and may be formed with, for example, a slit in a line shape or cross shape at its center portion.

The winding tapes 22 are positioned being separated from one another in a direction connecting the first covering portion 23 and the second covering portion 24, and one end portions of the winding tapes 22 are stuck on the cover 20.

### <Configurations of Separator, etc.>

Next, configurations of a separator 30 and the like to be attached to the holder 3 will be described (see FIG. 1, FIG. 2, and FIG. 4).

The separator 30 is made attachable to or detachable from the holder 3 (see FIG. 4). The separator 30 is formed of a resin material and includes a mounting portion 31 that has a substantially disk shape and is to be attached to the holder 3, a penetrating portion 32 that protrudes from one surface of the mounting portion 31 in a thickness direction of the mounting portion 31, and a pair of engaging portions 33 that protrude from the other surface of the mounting portion 31 in the thickness direction.

The mounting portion 31 is formed having an outer diameter that is substantially the same in size as an outer diameter of the cylindrical coupling part 14 of the holder 3. A surface of the mounting portion 31 on the penetrating portion 32 side in the thickness direction is formed as a pressing surface 31a.

The penetrating portion 32 includes a joining portion 34 continuing from the mounting portion 31 and being formed in a rectangular block shape and includes an inserting protrusion portion 35 protruding from the joining portion 34 and has a shaft shape. A distal end portion of the inserting protrusion portion 35 is provided as a tearing protrusion portion 35a formed in a tapered shape. Note that the inserting protrusion portion 35 may be formed entirely in a tapered shape. However, by forming only the distal end portion of the inserting protrusion portion 35 in a tapered shape, it is possible to make the inserting protrusion portion 35 have high stiffness.

The inserting protrusion portion 35 is formed with a groove at its intermediate portion in a protruding direction of the inserting protrusion portion 35, and the groove is formed as a lockable portion 35b.

An adapter 36 is made attachable to or detachable from the separator 30 (see FIG. 1 and FIG. 2). The adapter 36 has a function of holding a surgical instrument 80.

The adapter 36 is formed by, for example, joining an upper case 37 and a lower case 38 in the up-down direction and is provided with one end portion as an attachable portion 36a that is to be attached to the separator 30 by engagement.

The surgical instrument 80 is provided as, for example, a scope unit with an endoscope and includes a shaft part 81 inside which a plurality of lenses are disposed, a camera head 82 that is joined to one end portion of the shaft part 81, and a light guide 83 that is joined to a middle portion of the shaft part 81. Inside the camera head 82, an imaging device not illustrated is disposed.

To the camera head 82, cables not illustrated that are a signal line and a power source line are connected, and to the light guide 83, a light guide cable not illustrated that guides light is connected.

A distal end portion of the shaft part 81 of the surgical instrument 80 is to be inserted into an inside of a body cavity 201 through the port 202 formed on the patient 200. With the distal end portion of the shaft part 81 being inserted into the inside of the body cavity 201, illumination light is emitted from the distal end portion of the shaft part 81, and the imaging device images a state of the inside of the body cavity 201.

The surgical instrument 80 is held by the adapter 36 by the camera head 82 being sandwiched between the upper case 37 and the lower case 38 in the up-down direction. The adapter 36 holding the surgical instrument 80 is attached to the separator 30 by the attachable portion 36a being engaged with the engaging portions 33. Therefore, the surgical instrument 80 is held by the holder 3 via the adapter 36 and the separator 30.

### <Covered State Brought about by Drape>

Next, a covered state brought about by the drape will be described (see FIG. 4, FIG. 8, and FIG. 9).

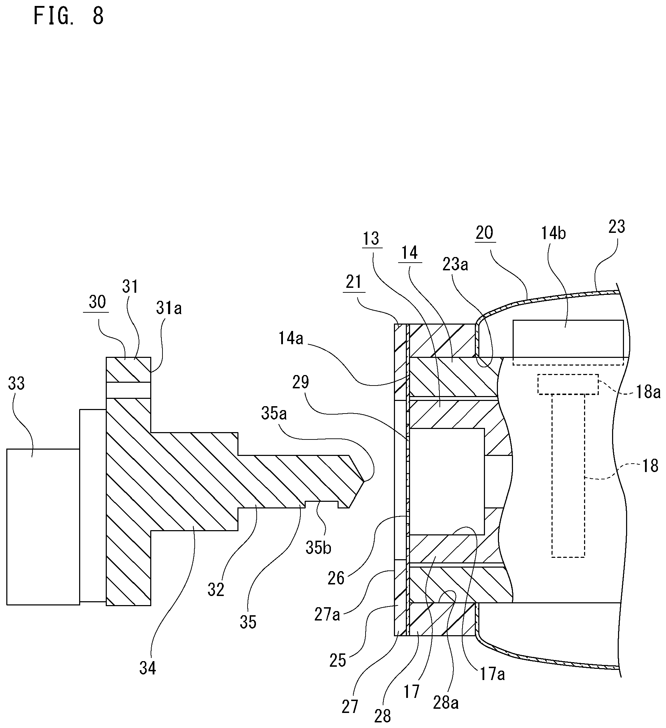

The drape 19 is put on the apparatus body 2 and the holder 3 (see FIG. 4). Therefore, being located within the cover 20, the apparatus body 2 and the holder 3 are covered with the drape 19. In a state where the apparatus body 2 and the holder 3 are covered with the drape 19, the hood 21 is attached to a distal end portion of the cylindrical coupling part 14 of the holder 3, bringing about a state where the closure sheet 26 of the hood 21 is in contact with the abutting surface 14a of the cylindrical coupling part 14, and the distal end portion of the cylindrical coupling part 14 is covered with the cylindrical portion 28 of the hood 21 from an outer circumferential side (see FIG. 8).

As seen from the above, since the drape 19 is provided with the hood 21 to be attached to the one end portion of the holder 3, the hood 21 serves as a guide for covering the apparatus body 2 and the holder 3 with the drape 19, and thus it is possible to cover the apparatus body 2 and the holder 3 with the drape 19 speedily and reliably.

In the state where the apparatus body 2 and the holder 3 are covered with the drape 19, the plurality of winding tapes 22 are wound around an outer surface of the cover 20 at their respective positions and tightened with fixtures or the like not illustrated (see FIG. 4). Therefore, the winding tapes 22 prevent the cover 20 from, for example, being turned up and thus keep the state where the apparatus body 2 and the holder 3 are covered with the drape 19.

In a state where the fixation base 4 of the apparatus body 2 is fixed to the fixing rail 100a of the operating table 100 with the clamper 8, for example, the entire fixation base 4 including the clamper 8 is covered with the drape 19 in a state where the fixation base 4 is fixed to the fixing rail 100a.

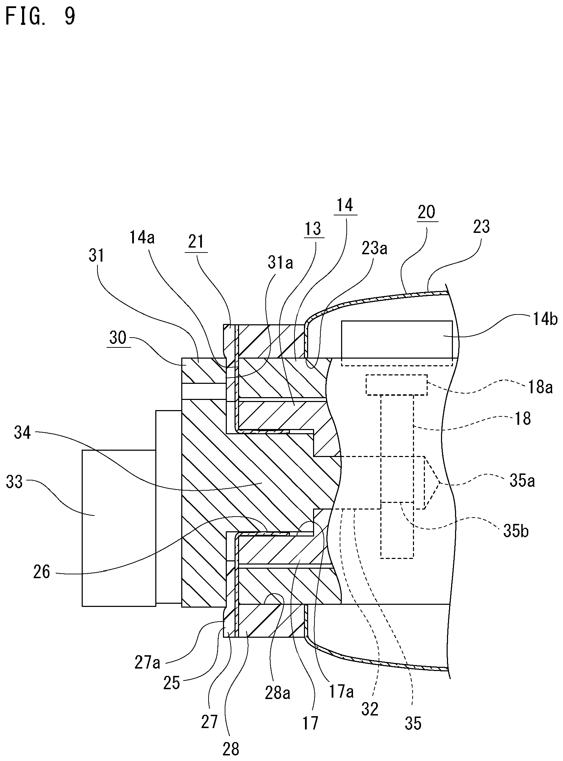

In the state where the apparatus body 2 and the holder 3 are covered with the drape 19, the separator 30 is attached to the holder 3 via the hood 21. When the separator 30 is attached to the holder 3 via the hood 21, the penetrating portion 32 of the separator 30 breaks through the closure sheet 26 of the hood 21, and the penetrating portion 32 penetrates into the penetration hole 28a of the hood 21 (see FIG. 9). Since the penetrating portion 32 is provided with the tearing protrusion portion 35a formed in a tapered shape, the tearing protrusion portion 35a forces open the closure sheet 26 and breaks through the closure sheet 26.

At this time, since the closure sheet 26 is formed with the perforations 29, the closure sheet 26 is torn from the perforations 29.

In the penetrating portion 32 penetrating into the penetration hole 28a of the hood 21, the joining portion 34 is inserted into the joint recess 17a of the detachable part 17 of the holder 3, and the inserting protrusion portion 35 is inserted into the insertion hole 15a of the shaft part 15 of the holder 3. Onto the lockable portion 35b of the inserting protrusion portion 35 being inserted into the insertion hole 15a, the lock mechanism 18 of the holder 3 is partially fitted, and the separator 30 is locked into the holder 3 by the lock mechanism 18. In a state where the separator 30 is locked into the holder 3, the mounting portion 31 is pressed against the pressable surface 27a of the surface portion 27 of the hood 21, and the penetration hole 28a is closed by the separator 30.

As seen from the above, since the separator 30 is locked into the holder 3 by the lock mechanism 18 in a state of being attached to the holder 3, unintended displacement of the separator 30 with respect to the holder 3 is restricted in a state where the separator 30 is attached to the holder 3, and thus a state where the penetration hole 28a is closed by the separator 30 can be reliably kept.

By operating the unlock button 14b of the holder 3 in the state where the separator 30 is locked into the holder 3 as described above, the lock mechanism 18 is caused to operate to unlock the separator 30, allowing the separator 30 to be detached from the holder 3.

### <Conclusion>

As described above, the hood 21 is provided with the closure sheet 26 that closes the penetration hole 28a in a state before the separator 30 is attached to the holder 3, and the separator 30 is attached to the holder 3 in a state where the penetrating portion 32 breaks through the closure sheet 26 to penetrate into the penetration hole 28a, and the penetration hole 28a is closed by the mounting portion 31.

Therefore, the penetration hole 28a of the hood 21 is closed by the closure sheet 26 in the state before the separator 30 is attached to the holder 3, and the penetration hole 28a is closed by the mounting portion 31 in the state where the separator 30 is attached to the holder 3, so that it is possible to keep a sanitary area that is an area where holder 3 and the apparatus body 2 covered with the drape 19 are present.

The outer circumferential portion of the closure sheet 26 is stuck on the mounting base 25 and held by the mounting base 25 under a certain tension, so that the closure sheet 26 is easily torn by the tearing protrusion portion 35a at a time when the separator 30 is attached to the holder 3, and thus it is possible to attach the separator 30 to the holder 3 easily with a weak force.

A portion to be broken through by the penetrating portion 32 is a portion inside the mounting base 25 of the hood 21, and the hood 21 serves as a guide to the portion to be broken through, which emphasizes a target portion to break through, and thus it is possible to attach the separator 30 to the holder 3 accurately and easily.

The distal end portion of the penetrating portion 32 is formed in a tapered shape as the tearing protrusion portion 35a, so that the penetrating portion 32 easily breaks through the closure sheet 26, and thus it is possible to attach the separator 30 to the holder 3 speedily and easily.

The hood 21 is formed with the perforations 29, so that the penetrating portion 32 easily breaks through the hood 21 from the perforations 29, and thus it is possible to attach the separator 30 to the holder 3 further speedily and easily.

The perforations 29 are formed in a cross shape with the first linear part 29a and the second linear part 29b, and the penetrating portion 32 breaks through the hood 21 from the perforations 29 in a cross shape, and thus it is possible to attach the separator 30 to the holder 3 further speedily and easily.

In the drape 19, the hood 21 is provided with the closure sheet 26 that closes the penetration hole 28a in the state before the separator 30 is attached to the holder 3, the closure sheet 26 is broken through by the penetrating portion 32 at a time when the separator 30 is attached to the holder 3, and the penetration hole 28a is closed by the mounting portion 31 in the state where the separator 30 is attached to the holder 3.

Therefore, the penetration hole 28a of the hood 21 is closed by the closure sheet 26 in the state before the separator 30 is attached to the holder 3, and the penetration hole 28a is closed by the mounting portion 31 in the state where the separator 30 is attached to the holder 3, so that it is possible to keep a sanitary area that is an area where holder 3 and the apparatus body 2 covered with the drape 19 are present.

### Reference Signs List

- 1: surgery assist apparatus
- 2: apparatus body
- 3: holder
- 9: first coupling arm
- 10: second coupling arm
- 11: third coupling arm
- 18: lock mechanism
- 19: drape
- 20: cover
- 21: hood
- 22: winding tape
- 26: closure sheet
- 28a: penetration hole
- 29: perforation
- 30: separator
- 80: surgical instrument

## Claims

1. A drape (19) to be used for a surgery assist apparatus (1),
wherein the surgery assist apparatus includes an apparatus body (2) including a plurality of coupling arms (9, 10, 11) and includes a holder (3) supported by the apparatus body (2), a separator (30) being made attachable to or detachable from the holder (3), the separator (30) including a mounting portion (31) and a penetrating portion (32), the holder (3) and the apparatus body (2) being covered with the drape (19) in an operation,
the drape (19) comprising:
a cover (20) and a hood (21), the cover (20) being in a film form, the hood (21) being to be attached to one end portion of the holder (3) and being formed with a penetration hole (28a), wherein
the hood (21) includes a closure sheet (26) that closes the penetration hole (28a) in a state before the separator (30) is attached to the holder (3), and
at a time when the separator (30) is attached to the holder (3), the closure sheet (26) is broken through by the penetrating portion (32), and the penetration hole (28a) is closed by the mounting portion (31) in a state where the separator (30) is attached to the holder (3),
wherein the hood (21) includes a mounting base (25) that is configured to be mounted on the one end portion of the holder (3) in its longitudinal direction,
**characterized in that**
the mounting base (25) includes a surface portion (27) having an annular shape and a cylindrical portion (28) having a cylindrical shape and space inside formed as the penetration hole (28a), wherein the surface portion (27) and the cylindrical portion (28) are joined together in a state of sandwiching the closure sheet (26) in an axial direction.

2. A drape attachment structure for a surgery assist apparatus (1), wherein the drape attachment structure includes
the drape according to claim 1,
the separator (30) including the mounting portion (31) and the penetrating portion (32), the mounting portion (31) being capable of being in close contact with the hood (21), the penetrating portion (32) protruding from the mounting portion (31), and
wherein the separator (30) is attached to the holder (3) in a state where the penetrating portion (32) breaks through the closure sheet (26) to penetrate into the penetration hole (28a), and the penetration hole (28a) is closed by the mounting portion (31).

3. The drape attachment structure for a surgery assist apparatus (1) according to claim 2, wherein
the cover (20) and the closure sheet (26) are formed of an identical material.

4. The drape attachment structure for a surgery assist apparatus (1) according to claim 2 or claim 3, wherein
at least a distal end portion of the penetrating portion (32) is formed in a tapered shape.

5. The drape attachment structure for a surgery assist apparatus (1) according to any one of claims 2- 4, wherein
the closure sheet (26) is formed with perforations (29).

6. The drape attachment structure for a surgery assist apparatus (1) according to claim 5, wherein
the perforations (29) are formed in a cross shape.

7. The drape attachment structure for a surgery assist apparatus (1) according to claim 5 or 6, wherein
the hood (21) comprises the closure sheet (26).

8. The drape attachment structure for a surgery assist apparatus (1) according to any one of claims 2- 7, wherein
the holder (3) of the surgery assist apparatus (1) includes a lock mechanism (18), and
the separator (30) is locked into the holder (3) by the lock mechanism (18) in a state where the separator (30) is attached to the holder (3).

9. The drape attachment structure for a surgery assist apparatus (1) according to any one of claims 2- 8,
wherein
a surface of the surface portion (27) is formed as a pressable surface (27a) and a surface of the mounting portion (31) on the penetrating portion (32) side in the thickness direction is formed as a pressing surface (31a).

10. The drape attachment structure for a surgery assist apparatus (1) according to according to any one of claims 2- 9,
wherein
the mounting base (25) has elasticity.

## Patentansprüche

1. Drape (19) zur Verwendung in einer Chirurgie-Assistenzvorrichtung (1),
wobei die Chirurgie-Assistenzvorrichtung einen Vorrichtungskörper (2) einschließt, der eine Vielzahl von Kupplungsarmen (9, 10, 11) einschließt, und einen Halter (3), der vom Vorrichtungskörper getragen wird (2), einen Separator (30) einschließt, der ausgebildet ist, am Halter (3) befestigbar oder von ihm abnehmbar zu sein, wobei der Separator (30) einen Montageabschnitt (31) und einen Penetrationsabschnitt (32) einschließt, wobei der Halter (3) und der Vorrichtungskörper (2) bei einer Operation mit dem Drape (19) bedeckt sind,
wobei das Drape (19) enthält:
eine Abdeckung (20) und eine Haube (21), wobei die Abdeckung (20) in einer Folienform ist, die Haube (21) an einem Endabschnitt des Halters (3) befestigt ist und mit einem Penetrationsloch (28a) gebildet ist, wobei
die Haube (21) ein Verschluss-Blatt (26) einschließt, das das Penetrationsloch (28a) in einem Zustand verschließt, bevor der Separator (30) am Halter (3) befestigt ist, und
zu einem Zeitpunkt, in dem der Separator (30) am Halter (3) befestigt ist, ist das Verschluss-Blatt (26) durch den Penetrationsabschnitt (32) durchbrochen und ist das Penetrationsloch (28A) durch den Montageabschnitt (31) in einem Zustand verschlossen, in dem der Separator (30) am Halter (3) befestigt ist,
wobei die Haube (21) eine Montagebasis (25) einschließt, die konfiguriert ist, an einem Endabschnitt des Halters (3) in seiner Längsrichtung montiert zu werden,
**dadurch gekennzeichnet, dass**
die Montagebasis (25) einen Oberflächenabschnitt (27) mit einer ringförmigen Form und einen zylindrischen Abschnitt (28) mit einer zylindrischen Form und Raum im Inneren einschließt, der als das Penetrationsloch (28a) gebildet ist, wobei der Oberflächenabschnitt (27) und der zylindrische Abschnitt (28) in einem Zustand eines sandwichartigen Anordnens des Verschluss-Blatts (26) in einer axialen Richtung verbunden sind.

2. Drape-Befestigungsstruktur für eine Chirurgie-Assistenzvorrichtung (1), wobei die Drape-Befestigungsstruktur einschließt
das Drape gemäß Anspruch 1,
den Separator (30), der den Montageabschnitt (31) und den Penetrationsabschnitt (32) einschließt, wobei der Montageabschnitt (31) fähig ist, in engem Kontakt mit der Haube zu sein (21), wobei der Penetrationsabschnitt (32) von dem Montageabschnitt (31) vorspringt, und
wobei der Separator (30) am Halter (3) in einem Zustand befestigt ist, in dem der Penetrationsabschnitt (32) durch das Verschluss-Blatt (26) bricht, um in das Penetrationsloch (28a) einzudringen, und das Penetrationsloch (28a) durch den Montageabschnitt (31) verschlossen ist.

3. Drape-Befestigungsstruktur für eine Chirurgie-Assistenzvorrichtung (1) gemäß Anspruch 2, wobei
der Deckel (20) und das Verschluss-Blatt (26) aus einem identischem Material gebildet sind.

4. Drape-Befestigungsstruktur für eine Chirurgie-Assistenzvorrichtung (1) gemäß Anspruch 2 oder Anspruch 3, wobei
mindestens ein distaler Endabschnitt des Penetrationsabschnitts (32) in einer konischen Form gebildet ist.

5. Drape-Befestigungsstruktur für eine Chirurgie-Assistenzvorrichtung (1) gemäß einem der Ansprüche 2-4, wobei
das Verschluss-Blatt (26) mit Perforationen (29) gebildet ist.

6. Drape-Befestigungsstruktur für eine Chirurgie-Assistenzvorrichtung (1) gemäß Anspruch 5, wobei
die Perforationen (29) in einer Kreuzform gebildet sind.

7. Drape-Befestigungsstruktur für eine Chirurgie-Assistenzvorrichtung (1) gemäß Antrag 5 oder 6, wobei
die Haube (21) das Verschluss-Blatt (26) enthält.

8. Drape-Befestigungsstruktur für eine Chirurgie-Assistenzvorrichtung (1) gemäß einem der Ansprüche 2-7, wobei
der Halter (3) der Chirurgie-Assistenzvorrichtung (1) einen Verriegelungsmechanismus (18) einschließt, und
der Separator (30) durch den Verriegelungsmechanismus (18) im Halter (3) in einem Zustand verriegelt ist, in dem der Separator (30) am Halter (3) befestigt ist.

9. Drape-Befestigungsstruktur für eine Chirurgie-Assistenzvorrichtung (1) gemäß einem der Ansprüche 2-8, wobei
eine Oberfläche des Oberflächenabschnitts (27) als pressbare Oberfläche (27A) gebildet ist und eine Oberfläche des Montageabschnitts (31) auf der Seite des Penetrationsabschnitts (32) in Dickenrichtung als eine Pressoberfläche (31A) gebildet ist.

10. Drape-Befestigungsstruktur für eine Chirurgie-Assistenzvorrichtung (1) gemäß einem der Ansprüche 2-9, wobei
die Montagebasis (25) Elastizität aufweist.

## Revendications

1. Un drap (19) destiné être utilisé pour un appareil d'assistance chirurgicale (1), dans lequel l'appareil d'assistance chirurgicale comprend un corps d'appareil (2) comprenant une pluralité de bras de couplage (9, 10, 11) et comprend un support (3) soutenu par le corps de l'appareil (2), un séparateur (30) qui est formé d'être fixable au ou détachable du support (3), le séparateur (30) comprenant une partie de montage (31) et une partie de pénétration (32), le support (3) et le corps de l'appareil (2) étant recouverts par le drap (19) lors d'une opération,
le drap (19) comprenant :
un couvercle (20) et un capot (21), le couvercle (20) étant sous forme de film, le capot (21) devant être fixé à une extrémité du support (3) et formé avec un trou de pénétration (28a), dans lequel
le capot (21) comprend une feuille de fermeture (26) qui ferme le trou de pénétration (28A) dans un état avant que le séparateur (30) ne soit fixé au support (3), et
au moment où le séparateur (30) est fixé au support (3), la feuille de fermeture (26) est percée par la partie de pénétration (32), et le trou de pénétration (28A) est fermé par la partie de montage (31) dans un état où le séparateur (30) est fixé au support (3),
dans lequel le capot (21) comprend une base de montage (25) qui est configurée pour être montée à une extrémité du support (3) dans sa direction longitudinale,
caractérisée en ce
la base de montage (25) comprend une partie de surface (27) ayant une forme annulaire et une partie cylindrique (28) ayant une forme cylindrique et un espace intérieur formé comme le trou de pénétration (28A), dans lequel la partie de surface (27) et la partie cylindrique (28) sont reliées ensemble dans un état d'enserrement la feuille de fermeture (26) dans une direction axiale.

2. Une structure d'attache de drap pour un appareil d'assistance chirurgicale (1), dans laquelle la structure d'attache de drap comprend
le drap selon la revendication 1,
le séparateur (30) comprenant la partie de montage (31) et la partie de pénétration (32), la partie de montage (31) pouvant être en contact étroit avec le capot (21), la partie de pénétration (32) faisant saillie de la partie de montage (31), et
dans laquelle le séparateur (30) est attaché au support (3) dans un état où la partie de pénétration (32) traverse la feuille de fermeture (26) pour pénétrer dans le trou de pénétration (28a), et le trou de pénétration (28a) est fermé par la partie de montage (31).

3. La structure d'attache du drap pour un appareil d'assistance chirurgicale (1) selon la revendication 2, dans laquelle
le couvercle (20) et la feuille de fermeture (26) sont faits d'un matériau identique.

4. La structure d'attache du drap pour un appareil d'assistance chirurgicale (1) selon la revendication 2 ou la revendication 3, dans laquelle
au moins une partie distale de la partie de pénétration (32) est formée en forme effilée.

5. La structure d'attache du drap pour un appareil d'assistance chirurgicale (1) selon l'une quelconque des revendications 2-4, dans laquelle
la feuille de fermeture (26) est formée avec des perforations (29).

6. La structure d'attache du drap pour un appareil d'assistance chirurgicale (1) selon la revendication 5, dans laquelle
les perforations (29) sont formées en forme de croix.

7. La structure d'attache du drap pour un appareil d'assistance chirurgicale (1) selon la revendication 5 ou 6, dans laquelle
le capot (21) contient la feuille de fermeture (26).

8. La structure d'attache du drap pour un appareil d'assistance chirurgicale (1) selon l'une quelconque des revendications 2-7, dans laquelle
le support (3) de l'appareil d'assistance chirurgicale (1) comprend un mécanisme de verrouillage (18), et
le séparateur (30) est verrouillé dans le support (3) par le mécanisme de verrouillage (18) dans un état où le séparateur (30) est attaché au support (3).

9. La structure d'attache du drap pour un appareil d'assistance chirurgicale (1) selon l'une quelconque des revendications 2-8, dans laquelle
une surface de la partie de surface (27) est formée comme une surface pressable (27A) et une surface de la partie de montage (31) au côté de la partie de pénétration (32) dans la direction d'épaisseur est formée comme une surface de pression (31A).

10. La structure d'attache du drap pour un appareil d'assistance chirurgicale (1) selon l'une quelconque des revendications 2-9, dans laquelle
la base de montage (25) possède une élasticité.
